# EUROPEAN PATENT APPLICATION

(11) **EP 4 230 232 A1**
(43) Date of publication of application: **23.08.2023**
(21) Application number: 22156925.4
(22) Date of filing: 16.02.2022
(51) Int. Cl.: A61L 27/12, A61L 27/54

(54) **BINDING OF CALCIUM PHOSPHATE BIOCERAMIC AND EXOSOMES VIA ANNEXIN V MEDIATOR MOLECULE**

(71) Applicant: HACETTEPE ÜNIVERSITESI, Ankara (TR); Sabanci Universitesi Nanoteknoloji Arastirma Ve Uygulama Merkezi, Istanbul (TR)
(72) Inventor: CIFTCI DEDE, Eda, ANKARA (TR); KORKUSUZ, Petek, ANKARA (TR); KORKUSUZ, Feza, ANKARA (TR); BAKAN MISIRLIO LU, Feray, ANKARA (TR)
(74) Representative: Yalçiner Patent and Consulting Ltd.

(57) **Abstract**

The invention relates to a carrier complex carrying the exosomes obtained from the bone marrow-derived mesenchymal stem cells. This carrier complex contains nanometer-sized calcium deficient hydroxyapatite which has a strong osteoconductive effect when applied to the injured area of the bone, mesenchymal stem cell-derived exosome which has an osteoinductive effect, as well as Annexin V molecule which binds nano-sized calcium deficient hydroxyapatite (CDHA) and the exosome to each other.

## Description

### Field of the Invention

The present invention relates to the necrotic or traumatic bone injuries having a low potential of self-recovery and a high rate of morbidity and mortality, and to osteoporosis, a metabolic bone disease.

The invention is directed to the restoration of bone injuries, comprising in a dose-adjusted manner, the effects of a supercarrier molecule that will consist of, the components, a non-cellular, osteoinductive exosome that carries cellular cargo that will induce ossification and calcium phosphate with osteoconductive effects for bone regeneration. Accordingly, in the clinic, it is aimed to develop a method for prevention or treatment purposes that can be used in replacement operations as well as effective and non-cellular.

### Background of the Invention (Prior Art)

The bone tissue is a dynamic tissue that can regenerate itself completely. In metabolic diseases such as osteoporosis, osteonecrosis, or traumatic bone injuries, the cell-matrix structure and mineralization mechanism of the bone may be impaired. Osteoporosis which increases with the aging population progresses with bone mineral loss and results in fractures, and osteonecrosis which is caused by traumas or bone circulation disorders are skeletal diseases that lead to the highest morbidity and mortality in the world.

According to the report dated 2003 of the World Health Organization, osteoporosis affects more than 75 million people in Europe, Japan, and the United States, and causes 2.3 million fractures annually (Durnell-Scguiling et al., 2011). In such fractures, it is required external and internal detections to be made as well as the reconstructive (osteoconductive) or inductive/constructive (osteoinductive) medical materials enabling the fracture recovery to be used. In the United States, approximately 20.000-30.000 people are diagnosed with osteonecrosis each year (Moya-Angeler et al., 2015) and it is reported that approximately 10% of all hip joint replacement surgeries are due to osteonecrosis (Mankin et al., 1992). Halting the progression of osteoporosis and osteonecrosis, which are common in the community, is important since a successful treatment effected by providing re-ossification and mineralization in the damaged area will reduce high morbidity and mortality rates.

In the bone diseases such as osteoporosis, in order to ensure the regeneration balance in necrotic bone injuries, medical treatment methods are applied first. These methods include nutritional supplements, medicines, exercises, and physical therapy approaches.

In the osteonecrosis treatment, the interventions protecting the femoral head and changing the surface of the femoral head or sacrificing the femoral head, and also as an alternative to these approaches, the operations for restoration of the injured area with cellular treatment methods and bioengineering approaches are performed. It is important to induce osteogenesis in osteoporosis treatment. For this purpose, in the clinic, the supportive cell therapies with the medication stimulating the functions of osteoblasts or preventing osteoclastic resorption are applied primarily (Antebi et al., 2014; Liu et al., 2015). Surgical treatment methods including joint replacement and fixation are applied for fractures due to osteoporosis (Pesce et al., 2009; Kim et al., 2017). At this stage, the autografts and allografts which are tissue patches of biological origin or synthetic biomaterials that are metals, ceramics, polymers, and composites thereof are applied (Korkusuz et al., 2011).

Calcium phosphate compounds are known to have high osteoconductive effects (Denry et al., 2016; Ginebra et al., 2018). In the treatment of traumatic or metabolic bone injuries that have reached the surgical stage, hydroxyapatite (HAp) which is the main component of the inorganic matrix of bone, and other synthetic calcium phosphate compounds can be used as implant coatings (Habraken et al., 2016; Kankιlιç et al., 2017; Li et al., 2018) or bone fillers (Korkusuz et al., 1999; Saikia et al., 2008; Habraken et al., 2016; Karr et al., 2018; Ginebra et al., 2018). It is known that the use of hydroxyapatite in nanometer size increases the material surface area (Korkusuz et al., 2013) and stiffness (Heimbach et al., 2017). In many osteoporosis studies, including pre-clinical and clinical, hydroxyapatite is used together with agents such as bisphosphonates (Kettenberger et al., 2015; Shen et al., 2016; Ma et al., 2016; Salamanna et al., 2019), strontium (Cheung et al., 2005; Tao et al., 2016; Chandran et al. 2016; Neves et al., 2016; Panzavolta et al., 2018; Zhao et al., 2020; Zhao et al., 2020-2), zoledronic acid (Khajuria et al., 2015; Khajuria et al., 2017) and ossein (Rüegsegger et al., 1995; Ciria-Recasens et al., 2011; Castelo-Branc et al., 2015; Castelo-Branc et al., 2020), which increase bone formation and reduce bone resorption.

In the clinic, it is shown that the hydroxyapatite-coated implants increase bone fixation (Moroni et al., 2001), reduce fixation errors in osteoporotic trochanteric fractures (Moroni et al., 2004), decrease periprosthetic bone loss in hip replacements of osteoporotic patients, and increase bone mineral density in the femoral stem of the implant (Rahmy et al., 2004).

However, in order to provide regeneration in critical bone losses, the effects of only osteoconductive calcium phosphates may be insufficient and it may need to be supported by osteoinductive-based approaches (Allais et al., 2015; Chen et al., 2015; Huettner et al. 2018). For this purpose, various growth factors (Casarrubios et al., 2020; Izquierdo-Barba et al., 2019; Wang et al., 2017; Shu et al., 2017) or stem and progenitor cells (Garcia-Gareta et al., 2015; Oryan et al., 2018) that provide them can be applied together with calcium phosphate compounds.

In pre-clinic osteoporosis studies, it is observed that mesenchymal stem cells (MSCs) derived from adipose (Chandaran et al., 2018) and the bone marrow-derived mesenchymal stem cells (Liu et al., 2016) with osteoprotegerin which are applied in the hydroxyapatite cell scaffold induce osteoporotic bone regeneration in high rates compared to only hydroxyapatite and healthy groups.

In a pre-clinic study for repairing jaw osteonecrosis due to bisphosphonate in rabbits, it is observed that complex of hydroxyapatite and mesenchymal stem cell derived from adipose increases new bone formation compared to only hydroxyapatite and healthy groups (Zang et al., 2019). In osteonecrosis clinical studies, it has been reported that the combination of associated porous hydroxyapatite and mononuclear cells derived from bone marrow is effective in reducing the osteonecrotic region (Yamasaki et al., 2010; Liu et al., 2012).

In cases where the bone is unable to recover itself, the osteoporosis causes fractures or necrotic bone losses in critical scale is observed; in order to provide regeneration, in conditions where only osteoconductive calcium phosphates are insufficient, more effective results are achieved by combining with osteoinductive based approaches (Allais et al., 2015; Chen et al., 2015; Huettner et al. 2018). Therefore, various growth factors (Wang et al., 2017; Shu et al., 2017) or stem and progenitor cells (Garcia-Gareta et al., 2015; Oryan et al., 2018) that provide them can be applied together with calcium phosphate compounds. Calcium and phosphate-based materials dissociating into Ca²⁺ and PO₄³⁻ ions by dissolving taken into cells through various channels (CaSR, L-type voltage-gated Ca channel, SLC20α1-phosphate carriers, etc.) and induce osteogenesis by affecting signal pathways such as Runx2, ERK1/2, BMP-2 (Shih et al., 2014; Jung et al., 2010; Khoshniat et al., 2011). The genes and proteins markers that can be interpreted with these pathways being active can be listed as BMP-2, BMP-4, BMP-7, Runx2/Cbfa-1, Osterix, type 1 collagen, osteopontin, osteocalcin, bone sialoproteins (BSP), and alkaline phosphatase (ALP). These genes and proteins control the functions and differentiation of the cells during bone formation and recovery processes (Tang et al., 2018).

Mesenchymal stem cells present a regenerative potential in the injured area by differentiating into mature cells or via the effect of the osteogenic, anti-inflammatory and immunosuppressive molecules they secrete as paracrine into the micro-environment in which they are situated (von Bahr et al., 2012; Wang 2018). It is reported that mesenchymal stem cells show their paracrine effects releasing intracellular vesicles called exosomes en masse out of the cell (Lou et al., 2017; Wu et al., 2017).

Exosomes are nanometer-sized intracellular vesicles that contain nucleic acids, lipids, and proteins of the cell from which they are obtained (Farooqi et al., 2018; Raposo et al. 2013). Mesenchymal stem cell exosomes are used in studies targeting bone regeneration since they contain molecules responsible for the regenerative effect processes of these cells (Deng et al., 2018; Marote et al., 2016), are easily taken into the target cell (Cheng et al., 2017; van Neil et al., 2018) and do not cause an immune response (Cosenza et al., 2018; Burello et al., 2016). Also, exosomes are preferred over MSCs because they can regulate transcription, provide gene expression control by means of the molecules they carry, and are not subjected to spontaneous malignant transformation since they do not contain nuclei (Rezaie et al., 2018).

It is proven that calcium phosphate cell scaffolds have positive effects on the ossification process via the PI3K/Akt pathway with MSC exosomes differentiated from human-induced pluripotent stem cells (Zhang et al., 2018).

In the study of Shuo Yang et al., at first hydrogels (HA-ALG) are synthesized, then hydroxyapatite (HAP) is added, thereby the in situ cross-linked embedded hyaluronic acid-alginate (HA-ALG) hydrogel system of injectable hydroxyapatite (HAP) is obtained. Finally, the integration of exosomes into in situ cross-linked embedded hyaluronic acid-alginate (HA-ALG) hydrogel system of hydroxyapatite (HAP) is provided.

Patent document numbered US 2018/0325958 A1 is based on compositions comprising a beneficial biological carrier and the products obtained from osteogenic precursor cells to stimulate bone formation in a subject. As examples of osteogenic progenitor cells, SM30, MEL2, and SKll cell lines; lysates, extracts, lyophilisates, exosome preparations and conditioned media as cell-derived preparations, and collagen (e.g. collagen sponges) and hydrogels as biological carriers are given.

In the prior art, since exosomes do not bind to the carrier with any binding molecule, it is difficult to follow the entry of the material into the cell and the amount of exosome usage at maximum efficiency cannot be determined and consequently, this causes material losses.

### Objects and Brief Description of the Invention

The present invention relates to a safe dose controlled treatment product that satisfies the above-mentioned requirements, resolve all of the disadvantages, and provides some additional advantages, presents a restorative and preventive effect in accordance with the disease profile in metabolic bone diseases and bone injuries having a high rate of morbidity and mortality by binding nano-sized calcium deficient hydroxyapatite (CDHA) and exosomes to each other via Annexin V mediator molecule, as well as to a method for obtaining the same.

The primary object of the invention is to obtain a treatment product complex by binding nano-sized calcium deficient hydroxyapatite (CDHA) and exosomes to each other via Annexin V mediator molecule and to detect the simultaneous and combined effects of the product by taking it into the cell together.

The object of the invention is to obtain a safe dose controlled treatment product usable in clinics practically that presents a restorative and preventive effect in accordance with the disease profile in metabolic bone diseases and bone injuries having a high rate of morbidity and mortality.

With the invention, the production of an innovative treatment agent with a potential effect, that does not pose the risks that come with the cellular treatments, have a dose control, and carry both osteoconductive and osteoinductive components which are necessary for bone regeneration is aimed.

Another object of the invention is to obtain a material that gives results quickly by penetrating the bone on the inside, supports the renewal of the bone tissue lost in osteoporosis by strengthening the bone, and that can be applied to the bone marrow cavity.

With the invention, following the material by providing entry of the material as a complex (CDHA and exosome together) into the cell is more comfortable rather than taking individual materials into the cell as it is in the prior art.

With the invention, it is possible to bind not only the injectable materials but also exosomes to CDHA-based tissue scaffolds; thereby the complex materials can be prepared in many forms in line with the requirement.

The carrier complex of the invention is used in conditions where the bone is needed to be protected and regenerated.

Further, the carrier complex of the invention is used in the treatment of necrotic or traumatic bone injuries, or in osteoporosis or osteonecrosis which are metabolic bone diseases.

Also, with the invention, the effective and safe dose range of nano-hydroxyapatite and mesenchymal stem cell exosomes in osteoblast cell lines is determined.

### Description of the Figures

The figures and related descriptions necessary for the subject matter of the invention to be understood better are given below.
**Figure 1: (A)** XRD diffractogram of CDHA sample dried by vacuum after the synthesis **(B)** XRD diffractogram of β-TCP sample sintered at 750°C after the synthesis are shown.
**Figure 2****:** XRD diffractograms of **(A)** CDHA after drying and **(B)** β-TCP samples after sintering at 750°C and XRD diffractograms of reference cards for HA (PDF No: 09-432) and β-TCP (PDF No: 09-169) are shown.
**Figure 3****:** Raman spectrums of CDHA after drying and β-TCP samples obtained after sintering at 750°C. **(A)** represents CDHA, **(B)** represents β-TCP In the spectrums peak 962 is an HA-specific peak, and peaks 947 and 969 are β-TCP-specific peaks.
**Figure 4****:** Bright-field transmission electron micrographs of **(A)** dried CDHA and **(B)** β-TCP samples obtained after sintering at 750°C are shown.
**Figure 5****:** The needle-like shape of mesenchymal stem cells attaching to the culture plate under the inverted light microscope is shown (×100, ×200, respectively).
**Figure 6****:** It is seen that human bone marrow-derived mesenchymal stem cells have a low ratio of CD38, HLA-DR, CD45 surface markers and a high ratio of CD29, CD44, CD73, and CD90 markers by flow cytometry technique. The vertical column shows the percentage of cells containing surface markers.
**Figure 7****:** The mean-standard deviation graph of the ELISA optical density values of human bone marrow-derived mesenchymal stem cells of osteogenic differentiation experiment at day 21 is shown.
**Figure 8****:** A box diagram graph of the expression data of SOX9 and COMP genes at day 7 of mesenchymal stem cells cultured in growth medium and chondrogenic differentiation medium is shown. In the graph, the horizontal axis indicates the medium type, while the vertical axis expresses the gene expression levels. Plain boxes represent SOX 9, while dashed boxes represent COMP gene expression.
**Figure 9****:** The distribution of exosome diameters is shown.
**Figure 10****:** The expression rates of exosomes captured by CD63 beads and stained with CD81 are shown. The peaks of the unstained group, the isotype group, and the CD81-linked groups are seen from top to bottom, respectively. It is determined that the mean fluorescence intensity of CD81 staining (387) is higher than that of the unstained (57) and isotype (213) groups.
**Figure 11****:** Exosomes **(A)** stained with uranyl acetate (×100000); **(B)** stained with phosphotungstic acid (x80000) are shown.
**Figure 12****:** Scanning electron micrographs of exosomes are shown. Exosomes are imaged at both **(A)** low magnification (x75000) and **(B)** high magnification (×150000). It is determined that the measurements of exosomes on micrographs are between 30 and 150 nm in size.
**Figure 13****:** The interaction of CDHA and Annexin V-CDHA together is shown by transmission electron micrographs. **(A)** CDHA ceramics (x20000); **(B)** CDHA gathered with Annexin V (×1500) are shown.
**Figure 14****:** Scanning electron micrograph of the complex material obtained by adding isolated exosomes to the Annexin V-CDHA mixture (×150000).
**Figure 15****:** Raman spectroscopy evaluation of complex materials and components is shown with Intensity/Wavenumber graphics.
**Figure 16****:** The positions of the bands of the complex materials and components obtained by using SDS-Page Gel electrophoresis on the gel are shown. Accordingly, the bands represent from left to right the Complex, CDHA-Annexin V, Annexin V, and Exosome groups, respectively.
**Figure 17****:** The time/concentration graph of the release of exosomes from the complex is shown. Depending on the time, it is seen that the exosomes are kept by the complex in the 1^{st} hour, then released in a controlled manner.

### Detailed Description of the Invention

In this detailed description, the operation steps for obtaining calcium deficient hydroxyapatite and human bone marrow-derived mesenchymal stem cells exosomes, binding the calcium deficient hydroxyapatites to exosomes via Annexin V and imaging them will be described only to a better understanding of the subject and is non-limiting at all.

The invention relates to a carrier complex carrying the exosomes obtained from the bone marrow-derived mesenchymal stem cells.

The carrier complex of the invention comprises;
a) Nano-hydroxyapatite having an osteoconductive effect,
b) Mesenchymal stem cell-derived exosome having an osteoinductive effect,
c) Annexin V molecule binding the nano-hydroxyapatite of step a and the exosome of step b to each other.

Nano-hydroxyapatite having an osteoconductive effect preferably has a needle-like structure. Further, its calcium/phosphate (Ca/P) molar ratio is less than 1.67.

The concentration of the nano-hydroxyapatite is 0.1-0.5 µg/ml and the concentration of the exosome is 25-100 µg/ml.

Annexin V molecule contains structurally binding sites of calcium and phosphatidylserine. When the amount of calcium in the environment increases or when Annexin V finds a Ca molecule to which it can bind, the phosphatidylserine binding sites on Annexin V are activated. Considering this mechanism, its binding to phosphatidylserine phospholipid in the membrane of exosomes is ensured via Annexin Vs attached to CDHA calcium.

### 1. Production and Characterization of Calcium Deficient Hydroxyapatites:

### 1.1. Production of Calcium Deficient Hydroxyapatites

Non-stoichiometric hydroxyapatites form the mineral part of the hard tissues in the human body. Since the Ca/P ratio of non-stoichiometric hydroxyapatites is less than 1.67, these compounds present a calcium deficiency. Therefore, these compounds are called calcium deficient hydroxyapatite (CDHA).

CDHA is synthesized by the wet precipitation method using high purity starting solutions of calcium and phosphorus. It has been reported in the literature that this reaction is very sensitive to reaction control parameters, especially pH change. This method is well known in art. The starting solutions used in the production thereof are given below.
- Calcium: calcium nitrate tetrahydrate (Ca(NO₃)₂.4H₂O) solution, pH≈5.5
- Phosphorus: ammonium dihydrogen phosphate (NH₄H₂PO₄) solution, pH≈5.5

In the preparation of both starting solutions 18.2 MQ.cm distilled water was used. Ca/P ratio for CDHA production is 1.55. Ammonia solution was used to adjust the pH of the starting solutions. In the reaction, the calcium starting solution was dropped into the phosphorus starting solution, which was being stirred at 45°C, in a controlled manner with a peristaltic pump. After the solutions were mixed, the formed gel was left to age at room temperature for 24 hours. At the end of the aging period, following the centrifugation process, the impure compounds remaining from the reaction medium were removed by washing with distilled water several times. The particles formed were dried at 80°C for 12 hours. CDHA obtained under these conditions subsequently is convertible to tricalcium phosphate (β-TCP) which is also a very important calcium phosphate compound with heat treatment. This phase is obtainable by a solid-state reaction of acidic anhydrous dicalcium phosphate with a base such as CaO or by wet chemical methods. Since the β-TCP is a high-temperature phase it cannot be obtained directly in a liquid phase, but it can be obtained from calcium deficient apatites that are non-stoichiometric and have a Ca/P ratio ranging between 1.33 to 1.65 after heat treatment. According to Equation 1, the conversion reaction of non-stoichiometric apatite to HA and β-TCP is given:

Ca₁₀₋ₓ (HPO₄) ₓ (PO₄)₆₋ₓ (OH)₂₋ₓ → Ca₁₀ (PO₄)₆ (OH)2 + β-Ca₃ (PO₄)₂ + xH₂O **(1)**

Ca₉(HPO₄)(PO₄)₅(OH)→3Ca₃(PO₄)₂+H₂O **(2)**

In the first equation, when x=1 and the Ca/P ratio is 1.5, the obtained apatite is called calcium deficient apatite (CDHA) and is chemically similar to the β-TCP phase. A pure β-TCP phase can be obtained from CDHA after heat treatment.

For this purpose, heat treatment was applied to obtain the β-TCP phase from the synthesized CDHA particles after drying.

### 1.2. Characterization of Calcium-Deficient Hydroxyapatites:

X-ray diffraction (XRD) and Raman Spectroscopy techniques were used to determine the structure of the obtained nanoparticle, and Transmission Electron Microscopy (TEM) techniques were used to determine the morphology of the particles. Raman Spectroscopy technique was also used to acquire qualitative and quantitative information about the bonds of organic and inorganic substances, as well as information about the related functional groups that constitute the substance or material structure.

### • X-Ray Diffraction and Raman Analysis:

Bruker D2 Phaser X-ray Diffractometer from Sabanci University Faculty of Engineering and Natural Sciences was used for XRD analysis. Renishaw Raman In Via device with 532 nm green laser attachment from Sabanci University Nanotechnology Research and Application Center was used for Raman Spectroscopy Analysis.

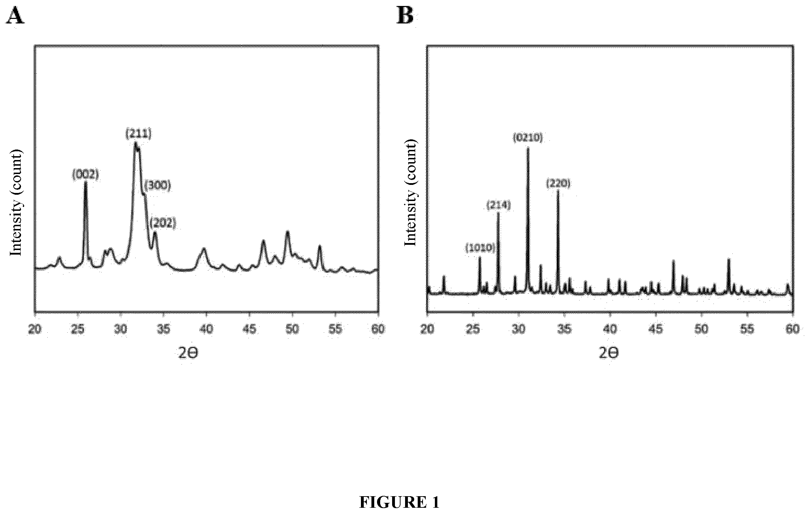

The XRD diffractogram of the vacuum-dried sample after synthesis is given in Figure 1-A. In phase analysis, a standard PDF (Powder Diffraction File) card numbered 009-0432 was used for HA, and characteristic peaks of HA were detected in the unsintered sample. There is no secondary phase in the diffractogram, and the presence of pure HA is shown.

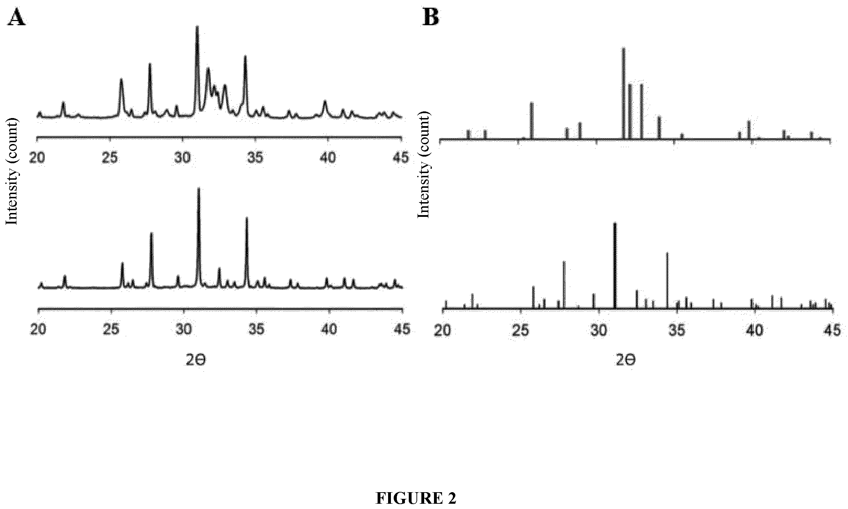

The XRD diffractogram of the sample sintered at 750°C after synthesis is given in Figure IB. In phase analysis, a standard PDF (Powder Diffraction File) card numbered 09-169 was used for the β-TCP phase, and it is seen that all CDHA is converted to β-TCP with the applied heat treatment in Figure 2.

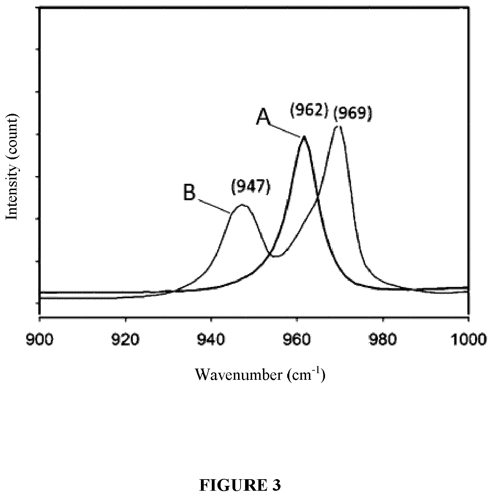

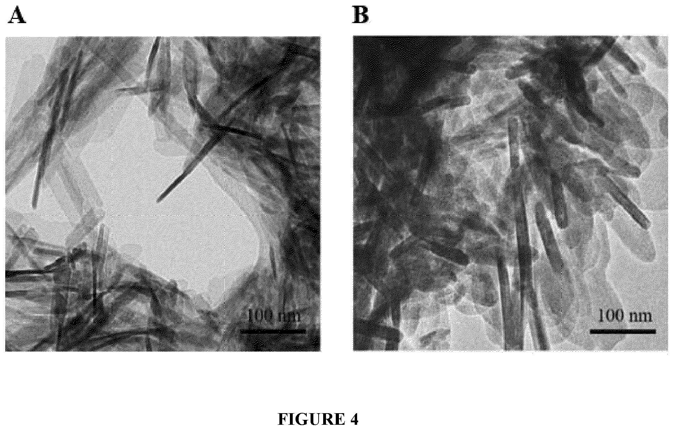

Raman spectra of the samples obtained after drying and sintering are given in Figure 3. Although the phases obtained with the XRD technique can be determined, the Raman spectroscopy technique can provide more precise information on the initiation and completion of the chemical conversion from CDHA to β-TCP at the molecular level. This conversion can be detected by observing the characteristic peak of the symmetric stretching mode (ν1) of the PO₄ group (P-O bond). This characteristic peak is observed at 962 cm⁻¹ for CDHA phase in the spectra in Figure 3, while they appear as two respective peaks observed at 947 and 969 cm⁻¹ with β-TCP formation after phase conversion. The main reason for that is that although there are 6 PO₄⁻³ groups in the HA unit cell, this number is 42 in the β-TCP phase. Therefore, in the β-TCP phase, the number of vibrations coming from the phosphate group is expected to be high. The spectra obtained in Figures 4-A and B support the transition to the β-TCP phase after heat treatment in parallel with the XRD results.

### • Morphological Analysis of Calcium Deficient Hydroxyapatite and Beta Tricalcium Phosphate by Transmission Electron Microscopy:

JEOL JEM-2100F UHR7HRP model high-resolution TEM device from Eski 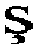 ehir Technical University was used for TEM analysis.

The bright-field TEM images of the dried samples after sintering are shown in Figures 4-A and B. It has been observed that the dried CDHA particles have a needle-like morphology similar to the morphology of stoichiometric or non-stoichiometric HA particles [16,20,23]. In the measurements performed, it was determined that the CDHA particles were approximately 20 nm in width and 150 nm in length. β-TCP particles obtained by phase conversion after sintering at 750°C have a very similar morphology to the CDHA particles. It was observed that the particles thickened only due to the applied heat treatment.

### 2. Isolation and Characterization of Exosomes from Human Bone Marrow-Derived Mesenchymal Stem Cells

### 2.1. Culture of Human Bone Marrow-Derived Mesenchymal Stem Cells:

In the first stage, the mesenchymal stem cells kept in the liquid nitrogen tank were dissolved under appropriate conditions. In order to do that, the cryo-vials were taken from the liquid nitrogen tank, quickly placed in a beaker containing water at 37°C, and left therein until no ice particles remained. Then, it was transferred to a 15 ml centrifuge tube with growth medium (MEM-α, 20% fetal bovine serum, 1% pen-strep, 1% L-glutamine) and centrifuged at 1200 rpm for 5 minutes. The obtained supernatant was discarded and the pellet was dissolved in 10 ml of growth medium. Cell count was performed by hemocytometric method. After counting, cells were planted in cell culture dishes by their amount, incubated in an incubator providing 37°C and 5% CO₂ conditions, and fed with a growth medium every 2-3 days. Characterization and exosome isolation steps were performed when mesenchymal stem cells reached ~80-90% density. The cells used in the experiments were used as the 5^{th} passage.

### 2.2. Characterization of Human Bone Marrow-Derived Mesenchymal Stem Cells:

According to the definition of The International Society for Cellular Therapy-ISCT the mesenchymal stem cells are characterized in that they express CD105, CD90, CD73 surface markers that present multipotent differentiation into at least two cell lines of mesoderm origin, such as fat, bone, and cartilage, as well as that can adhere to the container in which they are found under standard culture conditions, while do not express CD45, CD34, CD14, CD11b, CD79a or CD19 and HLA-DR surface markers. During the cell culture, the ability of the cells to adhere to the surface of the plastic cell culture dish and the polygonal, needle-shaped morphology which is a characteristic of mesenchymal stem cells were evaluated under an inverted light microscope.

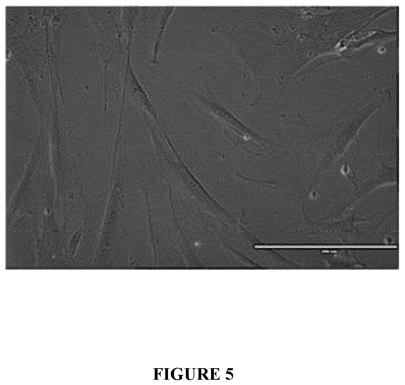

Bone marrow-derived mesenchymal stem cells were observed under the light microscope in the fourth passage, where they were attached to the surface of the culture dish. In Figure 5, needle-shaped mesenchymal stem cells are seen with their cytoplasmic extensions.

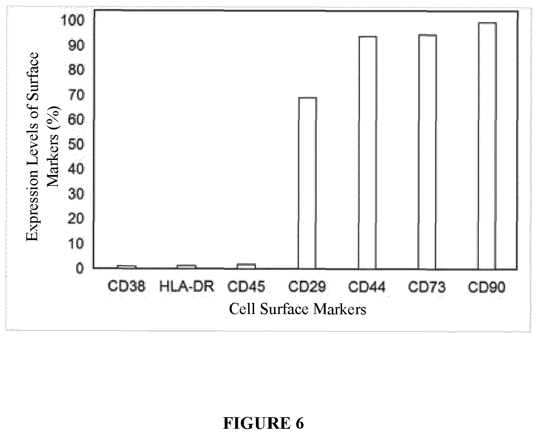

It was determined that the mesenchymal stem cells in the 4^{th} passage which were marked with the antibodies in Table 1 expressed CD38, HLA-DR, CD45 surface markers at low rates, while they express CD29, CD44, CD73 and CD90 surface markers at high rates (Figure 6). These data satisfy the conditions of being mesenchymal stem cell.

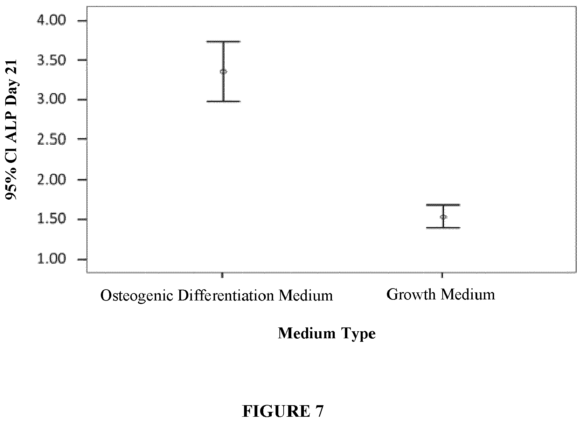

Osteogenic Differentiation; the intracellular ALP activity of mesenchymal stem cells (4^{th} passage) that were treated with osteogenic differentiation medium for twenty-one days was compared to intracellular ALP activity of mesenchymal stem cells that were treated with growth medium. As a result of the evaluation, it was determined that the mesenchymal stem cells, that were treated with osteogenic differentiation medium, gave statistically significant osteogenic differentiation values higher than the mesenchymal stem cells that were treated with growth medium (p= 0,001; Figure 7).

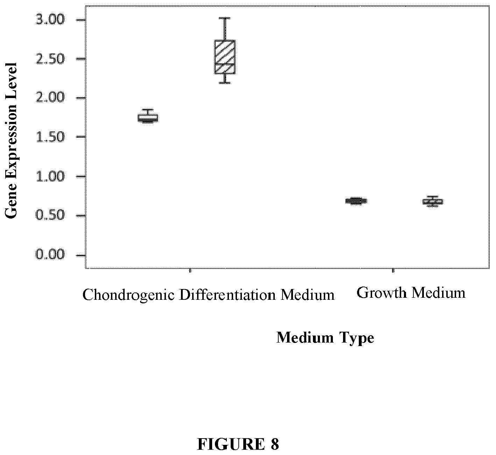

Chondrogenic Differentiation; At the end of seven days, it was determined that the expression of the chondrogenic differentiation marker SOX9 in mesenchymal stem cells treated with chondrogenic differentiation medium was higher than that in mesenchymal stem cells treated with growth medium (Figure 8).

**Table 1. The antibodies used in the invention are summarized in the table.**

| **Antibody** | **Fluorochrome** |
|---|---|
| Mouse anti-human CD73, IgGi | PE |
| Mouse anti-human CD44, IgGi | FITC |
| Mouse anti-human CD45, IgGi | FITC |
| Mouse anti-human CD90, IgGi | FITC |
| Mouse anti-human CD29, IgGi | APC |
| Mouse anti-human CD38, IgGi | PE |
| Mouse anti-human HLA-DR, IgG₂ | APC |

### 2.3. Extraction of Exosomes from Human Bone Marrow-derived Mesenchymal Stem Cells by Ultracentrifuge Method:

After the bone marrow-derived mesenchymal stem cells were grown under standard culture (37°C and 5% CO₂) conditions, the standard serum medium in which the cells were detected attaching to the container they were in by the light microscope was replaced with a medium containing serum from which exosomes were removed in order to prevent exosome contamination. The medium was collected after the cells whose densities reached 80% were incubated under cell culture conditions for one day. Then, exosome isolation was achieved by stepwise centrifugation methods. Stepwise centrifugation methods are as follows,
- 10 min at 400g (supernatant collected, pellet discarded)
- 10 min at 3000g (supernatant collected, pellet discarded)
- 10 min at 10000g (supernatant collected, pellet discarded)
- 30 min at 30000g (supernatant collected, pellet discarded)
- 90 min at 100000g (supernatant discarded, pellet collected)
- 90 min at 100000g (supernatant discarded, pellet collected)

The obtained pellet was diluted with HEPES or PBS in the required volume and the characterization stage was started.

### 2.4. Characterization of Exosomes Derived from Human Bone Marrow-Derived Mesenchymal Stem Cell:

Exosomes are characterized by protein quantification analysis, nanoparticle quantification and size analysis, flow cytometry, and transmission electron microscopy morphologically.

### • Quantitative Evaluation of Protein Amount:

For protein analysis, 10 different dilutions of Albumin solution were prepared and 20µl was added to a 96-well plate in two repetitions for the standard curve. The sample containing exosomes was diluted 1/20 and placed on a 96-well plate with 20µl in two repetitions. 80µl of BCA kit mixture was added thereto and incubated at 60°C for 30 minutes. After incubation, the plate was left to cool and then read at 462nm wavelength with an ELISA reader.

In the measurements performed for the samples containing exosomes, it was determined as a result of evaluations in two repetitions that there was an average of 1872,5 µg of protein per 1 ml. This amount was considered to be sufficient for characterization.

### • Nano-particle Quantity and Size Analysis:

The number and size of particles were determined by the Nanoparticle Tracking Analysis-NTA method with the iZON qNano Gold device (Izon Science Ltd, Christchurch, New Zealand). Exosome samples were diluted in 1:20 HEPES and the measurement was performed using the NP80 nanopore apparatus with a current of 0.56V targeting particles with a diameter of 40-225 nm.

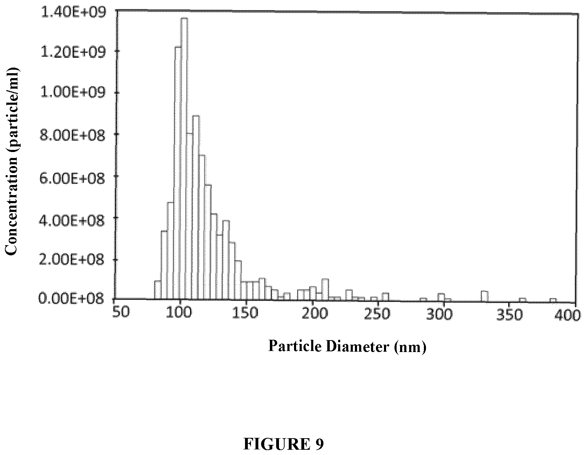

The mean particle diameter of the exosomes was determined as 121 nm (Figure 9). The maximum particle size range was found to be 80-383 nm. The mode of particle diameter is 96 nm. The particle concentration was determined as 5.34e+10 particles/ml.

### • Evaluation of Exosome Surface Markers by Flow Cytometry:

The amount of antibodies to be used was determined according to the amount of protein obtained. In order to evaluate the surface markers, at first, 5µl, 3,6 µm in size (4,2g/100ml) carboxyl latex beads (Thermo Fisher, MA, USA) were added to each tube determined for the marker according to each exosome isolation method. According to the determined protein amount, 1 µl of beads should be placed for each 1 µg of exosome. Beads are grouped such that they capture individual CD9, CD63, and CD81 markers. After the beads are added to the tubes, they will be left to mix in the rotator overnight. Then, the mixture was centrifuged at 10.000 g for 5 min and PBS was dissolved at 50 µl per pellet tube. After adding the relevant antibodies thereon, they were incubated at room temperature for 1 h in the dark and vortexed by adding 1 ml of PBS per tube. Afterward, the mixture was centrifuged at 10.000 g for 10 min, the obtained pellet was dissolved in 100 µl of PBS and vortexed. Prepared samples were placed in Novocyte brand flow cytometer device for analysis. The evaluation was performed by reading the surface markers of the cells marked with antibodies in 10.000 cases.

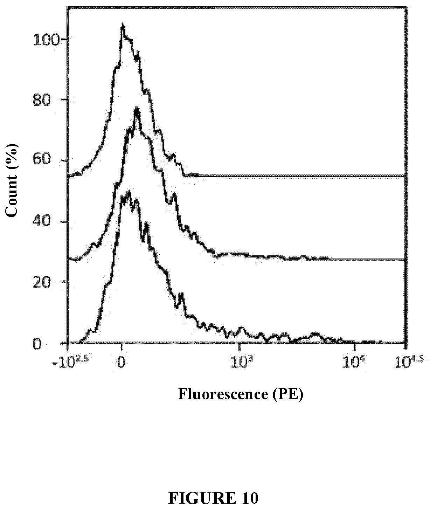

According to flow cytometry measurements, exosomes captured by CD81 were marked with CD63; that is, they were found to have both CD81 and CD63 surface markers (Figure 10). In the analysis, it was determined that CD81 staining was 1,82 times higher than the isotype. Accordingly, it was concluded that the exosomes were successfully obtained.

### • Electron Microscope Analysis of Exosomes:

Phosphotungstic acid (PTA) and uranyl acetate (UA) to be used in Transmission Electron Microscopy were prepared in advance. An appropriate amount (~5µl) of samples containing exosomes was dropped onto the matte surface of the grids, and the top of the grids was covered with the sample solution using a pipette tip. The petri dish was kept half-open for 10-15 minutes and the sample solutions on the grids were left to settle. Staining was carried out by droplet flotation method using PTA and UA filtered by means of a 0.22 filter. The grids were placed on the drops with their matte side with the exosome sample in contact with the droplet. The grids incubated for approximately 1-2 minutes in UA and PTA at room temperature were washed 3 times with distilled water in order to remove excess dye. After the washing steps, the grids were left to dry at room temperature. Drying grids were quantitatively examined at the ultrastructural level by means of a CCD camera (Gatan Inc., Pleasanton, CA, USA) with digital attachment at 80 kV (JEOL-JEM 1400, Japan) in TEM.

For Scanning Electron Microscopy, the exosome sample was diluted at 1:100 in HEPES. In this way, the formation of exosomes clusters is prevented. After the exosome sample was dropped on the silicon plate, it was dried in an oven at 30°C for 60 minutes and it is covered with 5nm thick gold (Cressington Sputter Coating-108 auto model, Ted Pella Inc., USA). The sample was examined by a secondary low vacuum electron detector (JIB-4601F; JEOL Ltd., Japan).

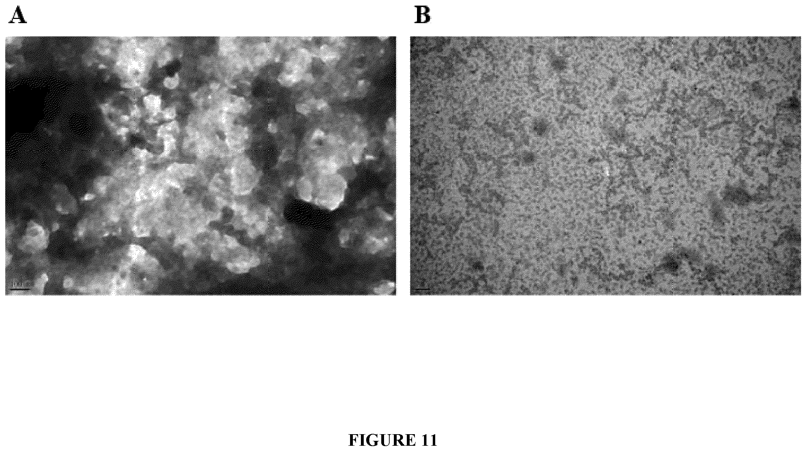

Exosomes were observed as round vesicles of 30-100 nanometers in size, individually or in clusters (Figure 11-A, B). Exosomes were detected as positively marked with uranyl acetate and negatively marked with phosphotungstic acid.

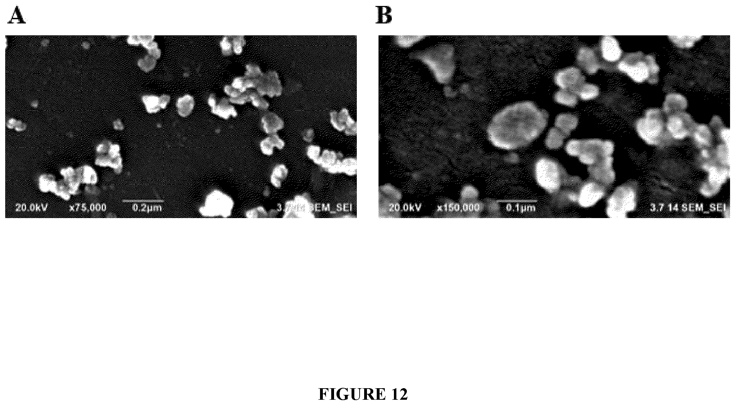

Exosomes are seen individually and in groups in scanning electron microscopy. The sizes of exosomes were observed between 30 and 150 nm (Figure 12A-B).

### 3. Binding of Calcium-Deficient Hydroxyapatites to Exosomes via Annexin V and Imaging of Binding

### 3.1. Modification of Calcium-Deficient Hydroxyapatites with Annexin V and Imaging

### • Modification of Calcium-Deficient Hydroxyapatites with Annexin V:

During the cell apoptosis, with increasing Ca molecules in the medium, Annexin V is used in the diagnosis of apoptosis by binding to phosphatidylserines which are normally present on the inner surfaces of the cell bilayer membrane but still at the same time rising to the membrane surface. Phosphotidylserine which is normally present on the inner surface of the cell bilayer membrane is found on the outer surface of the exosome membrane. The invention is differentiated by the use of Annexin V as a binding molecule. Annexin V has not been used as a binding molecule before. In the invention, CDHA nanoparticles are modified with Annexin V which will bind phosphatidylserine molecules in the membrane of the exosome by targeting. 2 groups were prepared for controlled analysis; (i) group containing CDHA only and (ii) group containing Annexin V and CDHA together. In the group containing only CDHA, 0,2 nanomolar CDHA solution prepared with HEPES was used. For the group containing Annexin V and CDHA the solutions were mixed in a 1:1 ratio.

### • Imaging of Modified Calcium-Deficient Hydroxyapatite:

After the samples were dropped on the silicon plate, they were dried in an oven at 30°C for 60 min. and covered with 5nm thick gold (Cressington Sputter Coating-108 auto model, Ted Pella Inc., USA). The sample was examined by a secondary low vacuum electron detector (JIB-4601F; JEOL Ltd., Japan).

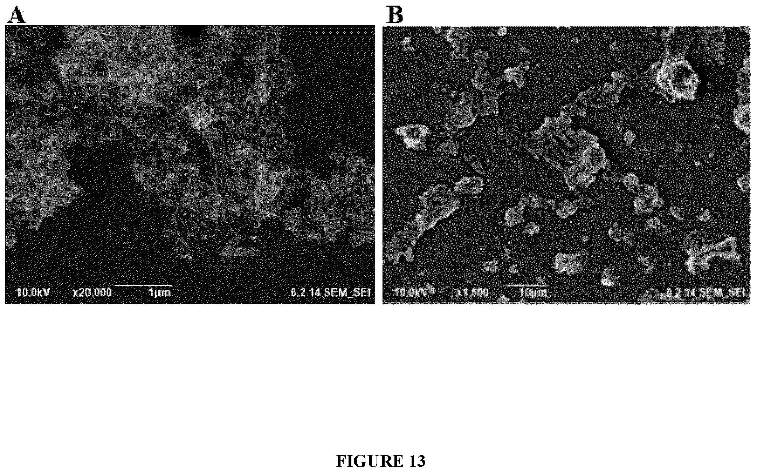

In the scanning electron micrographs, the crystalline structure in the CDHA group is seen (Figure 13-A). In the example where Annexin V and CDHA nanocrystals were applied together, it was observed that Annexin V and CDHA interacted by engaging each other (Figure 13-B).

### 3.2. Binding of Calcium-Deficient Hydroxyapatites to Exosomes via Annexin V and Imaging

### • Binding of Calcium-Deficient Hydroxyapatites to Exosomes via Annexin V

CDHA nanoceramics were able to target phosphatidylserine in the exosome membrane after being modified with Annexin V. The invention is differentiated by the use of Annexin V as a binding molecule. Annexin V has not been used as a binding molecule before.

### • Imaging of Binding of Calcium-Deficient Hydroxyapatites to Exosomes via Annexin V:

A high-resolution JEOL 4601F MultiBeam Platform (JEOL Ltd., Tokyo, Japan) device was used for the morphological evaluation of composite bonding. Before the morphological evaluation, the samples are covered with 5nm thick gold in order to reduce surface charge effects (Cressington Sputter Coating-108 auto model, Ted Pella Inc., USA). The sample was examined by a secondary low vacuum electron detector (JIB-4601F; JEOL Ltd., Japan). The samples were examined at x700 and ×150.000 magnification.

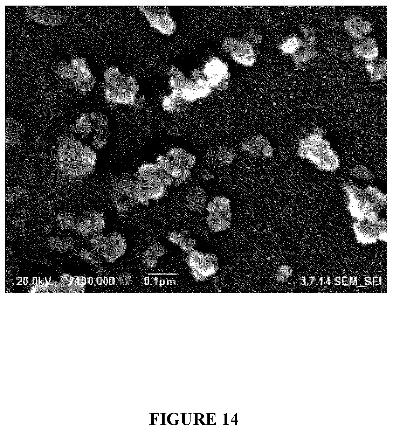

In the sample where Annexin V, CDHA nanocrystals, and exosomes were together, it was observed that the exosomes formed a complex with CDHA (Figure 14).

### 4. Binding analysis of the complex of calcium deficient hydroxyapatite-exosome

Raman spectroscopy, scanning electron microscopy, and SDS Page electrophoresis methods were used to demonstrate the binding of exosomes to calcium deficient hydroxyapatite via Annexin V.

### 4.1. Raman Spectroscopy Evaluation

Reinshaw Raman in Via spectrometer with 532 nm laser source and Leica light microscope (Leica, Vienna, Austria) was used in Raman spectroscopy evaluation. The device is set such that it collects data every 35 seconds using 30 µm laser power and display 10x and 20x magnifications.

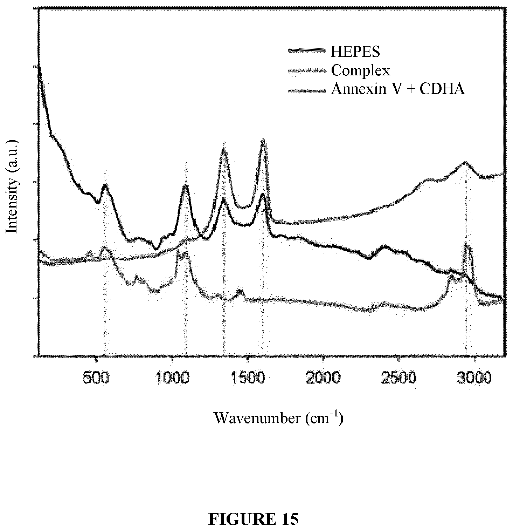

According to Raman spectroscopy evaluation; there are differences between samples (Figure 15). Here, when we compare the complex peak to other peaks, it is possible to mention a binding.

### 4.2. SDS Page Gel Electrophoresis

12% polyacrylamide gel (11 cm dissolved gel-0.75 mm thickness) containing copolymerized gelatin (1.5 mg/ml) was poured into the mold. The samples were mixed with a 1:1 loading solution (mixture containing SDS, bromophenol blue, glycerol, tris, and distilled water). The well was loaded with 20 ml of sample mixture for each. Electrophoresis was applied at 120V for 10 min, 190V for 60 min, and the samples were allowed to move on the gel.

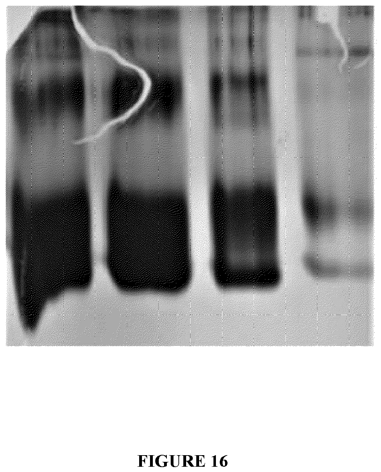

According to the result of electrophoresis, it was determined that the complex progressed in relation to the CDHA-Annexin V mixture and only the Annexin V sample (Figure 16). According to this result, it can be concluded that the binding has taken place. The bands were seen to be close since the weights of the materials were similar.

### 5. Release of exosomes from the complex of calcium deficient hydroxyapatite-exosome

According to the periods determined for release (0, 15, 30 minutes, 1, 2, 4, 8, 12, 24, 48 hours), the complexes were prepared in centrifuge tubes at equal concentrations. The expired sample was first centrifuged at 1200 rpm for 10 minutes, then the amount of exosomes contained in the samples taken from the supernatant was analyzed using a BCA protein determination method with an ELISA reader at a wavelength of 562 nm.

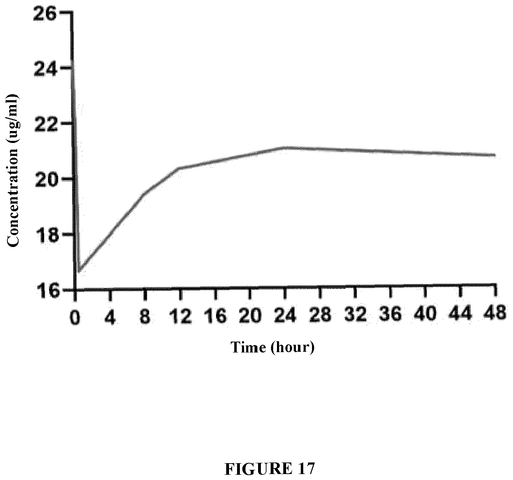

According to the release experiment, the decrease in the amount of exosomes starting from 25 µg in the first 1 hour shows that the exosomes bind with CDHA-Annexin V and release a large majority (approximately 10 µg) thereof in the first 12 hours (Figure 17). The release slowed down between 12-24 hours and it became stable at 21.04 µg after 24 hours. According to the results, it has been demonstrated that exosomes form complexes with CDHA-Annexin V and then their release occurs in a controlled manner.

## Claims

1. A carrier complex, **characterized in that** it comprises:
a) Nano-hydroxyapatite having an osteoconductive effect,
b) Mesenchymal stem cell-derived exosome having an osteoinductive effect,
c) Annexin V molecule binding the nano-hydroxyapatite of step a and the exosome of step b to each other.

2. The complex according to claim 1, **characterized in that** the hydroxyapatite has a needle-like structure.

3. The complex according to claim 1, **characterized in that** the calcium/phosphate (Ca/P) molar ratio of the hydroxyapatite is less than 1.67.

4. The carrier complex according to claim 1, **characterized in that** it is used in conditions where the bone is needed to be protected or regenerated.

5. The carrier complex according to claim 4, **characterized in that** it is used in the treatment of necrotic or traumatic bone injuries, or metabolic bone disease.

6. The carrier complex according to claim 5, **characterized in that** said bone disease is osteoporosis or osteonecrosis.

7. The complex according to claim 1, **characterized in that** the concentration of the nano-hydroxyapatite of step a is 0.1-0.5 µg/ml.

8. The complex according to claim 7, **characterized in that** the concentration of the exosome of step b is 25-100 µg/ml.
